(19) Europäisches Patentamt
European Patent Office
Office européen des brevets

(11) Veröffentlichungsnummer: **0 114 270**
**B1**

(12) **EUROPÄISCHE PATENTSCHRIFT**

(45) Veröffentlichungstag der Patentschrift: **01.04.87**

(51) Int. Cl.⁴: **C 07 D 493/04,** A 61 K 31/34 // (C07D493/04, 307:00, 307:00)

(21) Anmeldenummer: **83112218.9**

(22) Anmeldetag: **05.12.83**

(54) **Acylderivate von 1,4:3,6-Dianhydro-hexiten, Verfahren zu ihrer Herstellung und ihre Verwendung als Arzneimittel.**

(30) Priorität: **29.12.82 DE 3248548**

(43) Veröffentlichungstag der Anmeldung: **01.08.84 Patentblatt 84/31**

(45) Bekanntmachung des Hinweises auf die Patenterteilung: **01.04.87 Patentblatt 87/14**

(84) Benannte Vertragsstaaten: **AT BE CH DE FR GB IT LI LU NL SE**

(56) Entgegenhaltungen:
**EP - A - 0 061 055**
**EP - A - 0 065 267**
**EP - A - 0 070 401**
**DE - A - 2 117 571**
**DE - A - 2 935 451**
**DE - A - 2 938 464**
**US - A - 3 886 186**
**US - A - 4 065 488**

**CHEMICAL ABSTRACTS, Vol. 93, Nr. 3, 21. Juli 1980, Columbus, Ohio, USA GOODWIN, JAMES C.; HODGE, John E.; WEISLEDER DAVID "Preparation of bicyclic hexitol anhydrides by using acidic cation-exchange resin in a binary solvent, Carbon-13 NMR spectroscopy confirms configurational inversion in chloride displacement of methanesulfonate in isomannide and isosorbide derivatives" Seite 731, Spalte 2, Abstract-Nr. 26 679y**

(73) Patentinhaber: **Heinrich Mack Nachf., Postfach 140, D-7918 Illertissen (DE)**

(72) Erfinder: **Stoss, Peter, Dr. Dipl.-Chem., Mozartstrasse 15, D-7918 Illertissen (DE)**
Erfinder: **Leitold, Matyas, Dr., Klauflügelweg 21, D-7950 Biberach (DE)**

(74) Vertreter: **Lederer, Franz, Dr. et al, Vanderwerth, Lederer & Riederer Patentanwälte Lucile-Grahn-Strasse 22, D-8000 München 80 (DE)**

(56) Entgegenhaltungen: (Fortsetzung)

**Die Akte enthält technische Angaben, die nach dem Eingang der Anmeldung eingereicht wurden und die nicht in dieser Patentschrift enthalten sind.**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die vorliegende Erfindung betrifft neue Acylderivate von 1,4:3,6-Dianhydro-hexiten, Verfahren zu ihrer Herstellung sowie ihre Verwendung als Arzneimittel, insbesondere als herz- und kreislaufbeeinflussende Mittel.

Es ist bekannt, dass bestimmte 1,4:3,6-Dianhydro-hexite interessante pharmakologische Eigenschaften aufweisen. So ist für 1,4:3,6-Dianhydro-D-glucit (Isosorbid) eine diuretische Wirkung beschrieben, z.B. Proc. Soc. exp. Biol. Med. 119, 39 (1965), ebenso für 1,4:3,6-Dianhydro-D-manit (Isomannid), z.B. US-A 2 143 324. 1,4:3,6-Dianhydro-D-glucit-2,5-dinitrat (Isosorbid-dinitrat) sowie dessen Metabolit, Isosorbid-5-nitrat, sind bekannte Koronarvasodilatatoren, die therapeutisch genutzt werden.

Auch einige Acylderivate von 1,4:3,6-Dianhydro-hexiten wurden bereits pharmakologisch untersucht, z.B. in der DE-A 2 221 080, die u.a. Niederalkanoyl- und Benzoyl-Derivate der Isosorbid-mononitrate zum Inhalt hat und in der DE-A 3 028 289, wo u.a. Nicotinoyl-1,4:3,6-dianhydro-hexitnitrate beschrieben sind. Ferner wurden in der GB-A 1 027 891 Mono- und Di-nicotinoyl-1,4:3,6-dianhydro-hexite als kreislaufwirksam erkannt.

Gegenstand der vorliegenden Erfindung sind neue Acylderivate von 1,4:3,6-Dianhydro-hexiten der allgemeinen Formel I

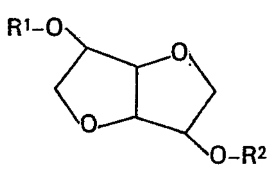

I

in welcher

$R^1$ für Wasserstoff, einen niederen Acylrest mit 2 bis 5 Kohlenstoffatomen, einen Pyridylcarbonylrest oder $NO_2$ steht und

$R^2$ für einen 1,4-Dihydropyridyl-carbonylrest der allgemeinen Formel II steht

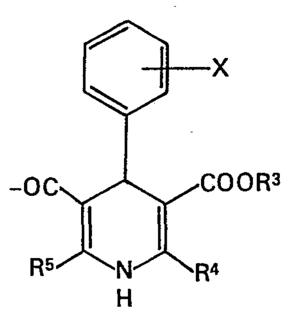

II

in welcher

X für Wasserstoff oder 1,2 oder 3 gleiche oder verschiedene Substituenten aus der Gruppe Alkoxy, Alkyl, Cyano, Dialkylamino, Halogen, Nitro oder Trifluormethyl oder für eine Methylendioxy-Gruppe steht,

$R^3$ einen geradkettigen oder verzweigten, gesättigten oder ungesättigten Kohlenwasserstoffrest mit 1 bis 5 Kohlenstoffatomen, wobei gegebenenfalls die Kette durch ein Sauerstoffatom unterbrochen und/oder der Kohlenwasserstoffrest gegebenenfalls durch eine Cyanogruppe substituiert sein kann, bedeutet.

$R^4$ und $R^5$ gleich oder verschieden sind und jeweils für eine niedere Alkylgruppe stehen,

sowie pharmazeutisch akzeptable Salze von zur Salzbildung befähigten Verbindungen.

Soweit nichts anderes angegeben, bedeuten Alkylgruppen auch in Gruppierungen wie Alkoxy und dergl. solche mit 1-5 C Atomen, vorzugsweise mit 1-3 C Atomen.

Zu den hier behandelten Acylderivaten der 1,4:3,6-Dianhydro-hexite gehören insbesondere die nachfolgend aufgeführten, stereoisomeren, durch Epimerisierung ineinander überführbaren Grundkörper, nämlich:

1,4:3,6-Dianhydro-L-idit (Isoidid) folgender Struktur,

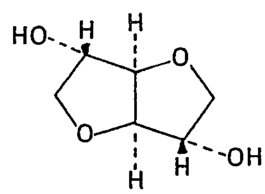

bei dem die OH-Gruppen in 2- und 5-Stellung jeweils exo-Konfiguration aufweisen, oder

1,4:3,6-Dianhydro-D-glucit (Isosorbid) folgender Struktur,

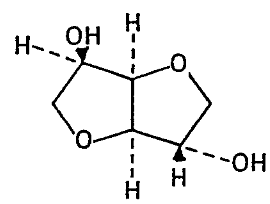

der eine 2-exo-ständige sowie eine 5-endo-ständige OH-Gruppe aufweist und dessen O-Derivate somit in zwei isomeren Formen auftreten können, oder

1,4:3,6-Dianhydro-D-mannit (Isomannid) folgender Struktur,

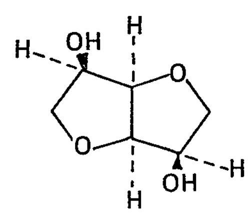

der zwei endo-ständige OH-Gruppen aufweist.

Insofern können die Reste $R^1$ und $R^2$ in der allgemeinen Formel I jeweils sowohl die 2- als auch die 5-Stellung besetzen.

Im Gegensatz zu den Glucit-Derivaten ist bei

den Idit- und Mannit-Derivaten eine Unterscheidung zwischen einer Substitution in der 2- und 5-Position nicht möglich. Eine kurze Zusammenfassung über die Stereochemie der 1,4:3,6-Dianhydro-hexite gibt J.A. Mills in Advances in Carbohydrate Chemistry 10, 1-53 (1955).

1,4:3,6-Dianhydro-hexite stellen optisch aktive Moleküle dar. Die Acylreste der allgemeinen Formel II besitzen im C-4 des 1,4-Dihydropyridin-Rings ein chirales Zentrum. Deshalb treten die erfindungsgemässen Verbindungen der allgemeinen Formel I in diastereoisomeren Formen auf, wobei für jeden Rest $R^1$ und ebenso für jeden Rest $R^2$ jeweils ein Diastereoisomerenpaar existiert. Sowohl die Diastereoisomerengemische als auch die getrennten, konfigurativ einheitlichen Komponenten sind Gegenstand dieser Erfindung.

Chirale 1,4-Dihydropyridin-carbonsäureester sind nach dem Stand der Technik bereits bekannt, z.B. DE-A 2 117 571, DE-A 2 549 568, DE-A 2 650 013 und DE-A 2 935 451. Darunter befinden sich auch solche, die in optisch aktive Antipoden aufgetrennt wurden. Jedoch liegen in keinem dieser vorbeschriebenen Fälle Derivate von 1,4:3,6-Dianhydro-hexiten vor.

Die hier erstmals vorgenommene intramolekulare Kombination des als pharmakophore Wirkgruppe bekannten 1,4-Dihydropyridyl-carbonyl-Restes mit der ebenfalls pharmakologisch aktiven Klasse der 1,4:3,6-Dianhydro-hexite ist neu. Sie gestattet eine weitere Wirkungsdifferenzierung, ermöglicht neue Einblicke in Struktur-Wirkungsbeziehungen, eröffnet zusätzliche Anwendungsgebiete und stellt somit eine Bereicherung der therapeutischen Möglichkeiten dar.

Besonders bevorzugte Verbindungen der allgemeinen Formel I sind solche, bei denen $R^1$ Wasserstoff bedeutet.

Eine Gruppe erfindungsgemässer Verbindungen umfasst solche, in welchen der 1,4:3,6-Dianhydrohexit Isosorbid ist, wobei das Sauerstoffatom in 2-Stellung (bzw. in 5-Stellung) substituiert ist mit $R^1$ und das Sauerstoffatom in der 5-Stellung (bzw. in 2-Stellung) substituiert ist mit $R^2$, wobei ausserdem vorzugsweise $R^3$, $R^4$ und $R^5$ jeweils Methyl bedeuten und $R^1$ Acetyl und X Wasserstoff, oder $R^1$ Nitro und X 3-Nitro, oder $R^1$ Nitro und X 4-Cyano, oder $R^1$ Wasserstoff und X Wasserstoff, oder $R^1$ Wasserstoff und X 3-Nitro, oder $R^1$ Butanoyl und X 2-Nitro, oder $R^1$ Nitro und X Wasserstoff, oder $R^1$ Acetyl und X 3-Nitro, oder $R^1$ Acetyl und X 2-Nitro, oder $R^1$ Wasserstoff und X 2-Nitro, oder $R^1$ Nitro und X 4-Fluoro, oder $R^1$ Nitro und X 3-Trifluoromethyl, oder $R^1$ Nitro und X 3,4-Methylendioxy, oder $R^1$ Nitro und X 2-Nitro bedeuten.

Eine andere Klasse erfindungsgemässer Verbindungen umfasst solche, in welchen der 1,4:3,6-Dianhydrohexit Isosorbid ist, wobei das Sauerstoffatom in der 2-Stellung (bzw. in 5-Stellung) substituiert ist mit $R^2$ und das Sauerstoffatom in der 5-Stellung (bzw. in 2-Stellung) substituiert ist mit $R^1$, vorzugsweise solche Verbindungen, bei denen $R^3$, $R^4$ und $R^5$ jeweils Methyl bedeuten und wobei ferner $R^2$ Nitro und X Wasserstoff, oder $R^2$ Nitro und X 3-Nitro, oder $R^2$ Wasserstoff und X 3-Nitro, oder $R^2$ Wasserstoff und X 2-Nitro, oder $R^2$ Acetyl und X 2-Nitro, oder $R^2$ Acetyl und X 3-Nitro, oder $R^2$ Nitro und X 3,4-Methylendioxy, oder $R^2$ Nitro und X 4-Fluoro, oder $R^2$ Nitro und X 2-Methoxy, oder $R^2$ Nitro und X 4-Cyano, oder $R^2$ Nitro und X 3-Trifluoromethyl bedeuten.

Bevorzugt sind auch Verbindungen in denen $R^2$ Nitro, X 3-Nitro, $R^3$ Butyl und $R^4$ und $R^5$ jeweils Methyl bedeuten; oder $R^2$ Nitro, X 3-Nitro, $R^3$ Isopropyl und $R^4$ und $R^5$ jeweils Methyl bedeuten; oder $R^2$ Nitro, X 3-Nitro, $R^3$ Ethyl und $R^4$ und $R^5$ jeweils Methyl bedeuten; oder $R^2$ Nitro, X 3-Nitro, $R^3$ Allyl und $R^4$ und $R^5$ jeweils Methyl bedeuten; oder $R^2$ Nitro, X 3-Nitro, $R^3$ Iso-butyl und $R^4$ und $R^5$ jeweils Methyl bedeuten; oder $R^2$ Nitro, X 3-Nitro, $R^3$ tertbutyl und $R^4$ und $R^5$ jeweils Methyl bedeuten; oder $R^2$ Nitro, X 3-Nitro, $R^3$ 3-Ethoxypropyl und $R^4$ und $R^5$ jeweils Methyl bedeuten.

Die Erfindung umfasst auch pharmazeutische Zubereitungen, die mindestens eine erfindungsgemässe Verbindung und einen pharmazeutisch annehmbaren Träger oder ein pharmazeutisch annehmbares Verdünnungsmittel enthalten. Die Erfindung betrifft ferner die Verwendung der erfindungsgemässen Verbindungen zur Bekämpfung kreislaufbedingter, insbesondere kardiovasculärer Erkrankungen sowie Heilverfahren zur Kontrolle kardiovasculärer Erkrankungen in Säugetieren, einschliesslich des Menschen, wobei das Lebewesen mit einer wirksamen Menge einer erfindungsgemässen Verbindung behandelt wird.

Es wurde gefunden, dass man die erfindungsgemässen Verbindungen erhält, wenn man

A) einen Aryliden-β-ketocarbonsäure-ester der allgemeinen Formel III

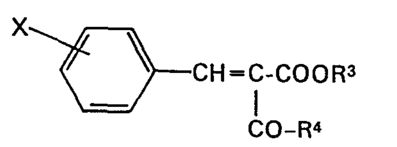

III

in der X, $R^3$ und $R^4$ die angegebene Bedeutung besitzen, in an sich bekannter Weise mit einem Enaminocarbonsäure-(1,4:3,6-dianhydro-hexit)-ester der allgemeinen Formel IV

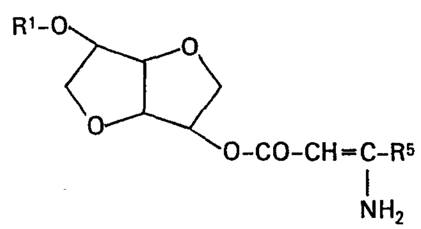

IV

in der $R^1$ und $R^5$ die angegebene Bedeutung besitzen, umsetzt oder

B) einen Aldehyd der allgemeinen Formel V

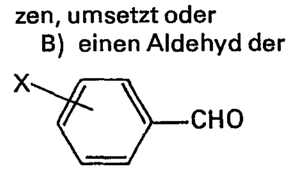

V

in der X die angegebene Bedeutung besitzt, in an sich bekannter Weise mit einem β-Ketocarbonsäure-ester der allgemeinen Formel VI

$R^4-CO-CH_2-COOR^3$ VI

in der $R^3$ und $R^4$ die angegebene Bedeutung besitzen und mit einem Enaminocarbonsäure-(1,4:3,6-dianhydro-hexit)-ester der allgemeinen Formel IV, in der $R^1$ und $R^5$ die angegebene Bedeutung haben, umsetzt, oder

C) einen Aldehyd der allgemeinen Formel V, in der X die angegebene Bedeutung besitzt, in an sich bekannter Weise, mit einem Enaminocarbonsäureester der allgemeinen Formel VII

$R^4-C=CH-COOR^3$ VII
|
$NH_2$

in der $R^3$ und $R^4$ die angegebene Bedeutung besitzen und mit einem β-Ketocarbonsäure-(1,4:3,6-dianhydro-hexit)-ester der allgemeinen Formel VIII

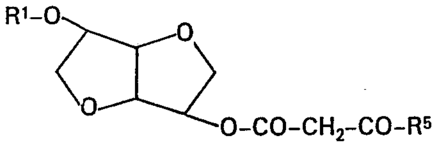

VIII

in der $R^1$ und $R^5$ die angegebene Bedeutung besitzen, umsetzt, oder

D) einen Aryliden-β-ketocarbonsäureester der allgemeinen Formel III, in der X, $R^3$ und $R^4$ die angegebene Bedeutung besitzen, in an sich bekannter Weise mit einem β-Ketocarbonsäure-(1,4:3,6-dianhydro-hexit)-ester der allgemeinen Formel VIII, in der $R^1$ und $R^5$ die angegebene Bedeutung besitzen, und mit Ammoniak umsetzt,

und gegebenenfalls ein so erhaltenes Acylderivat der allgemeinen Formel I in an sich bekannter Weise in ein Salz überführt.

Bei der Durchführung der Verfahren A) – D) werden die an der Reaktion beteiligten Stoffe jeweils etwa in molaren Mengen eingesetzt. Das verwendete Ammoniak wird zweckmässig im Überschuss zugegeben. Die Umsetzungen können sowohl ohne Lösungsmittel, als auch in Wasser oder in allen unter den Reaktionsbedingungen inerten organischen Lösungsmitteln vorgenommen werden. Dazu gehören vorzugsweise Alkohole wie Methanol, Ethanol, Propanol und Isopropanol oder Ether wie Diethylether, Dioxan, Tetrahydrofuran, Glykolmonomethylether und Glycoldimethylether oder Eisessig, Pyridin, Acetonitril, Dimethylformamid oder Dimethylsulfoxid.

Die Reaktionstemperaturen sind in weiten Bereichen variabel und liegen im allgemeinen zwischen 20 und 200°C. Vorzugsweise arbeitet man bei 50–120°C, insbesondere bei der Siedetemperatur des jeweiligen Lösungsmittels.

Die Umsetzungen können bei Normaldruck, aber auch bei erhöhtem Druck durchgeführt werden. Im allgemeinen arbeitet man bei Normaldruck.

Die Isolierung und Reinigung der erfindungsgemässen Verbindungen erfolgt auf übliche Weise. In einigen Fällen kristallisieren die Reaktionsprodukte nach beendeter Umsetzung direkt aus. In anderen Fällen ist es zweckmässig, das Lösungsmittel im Vakuum abzudestillieren, danach den Rückstand zur Kristallisation zu bringen und gegebenenfalls aus einem geeigneten Lösungsmittel umzukristallisieren.

Die Umsetzungen A) – D) sind an sich bekannt. Sie stellen Varianten der Hantzschen Pyridin-Synthese dar, wie sie teilweise bereits von Knoevenagel, Ber. Dtsch. Chem. Ges. 31, 738 (1898) beschrieben wurden. Neuere Zusammenfassungen finden sich z.B. in Arzneim.-Forsch. 31, 407 (1981), Angew. Chem. 93, 755 (1981), sowie Drugs of the Future VI, 427 (1981).

Die erfindungsgemäss verwendeten Ausgangsprodukte der allgemeinen Formeln IV und VIII sind neu, die anderen sind bereits bekannt oder können nach literaturbekannten Methoden hergestellt werden. Die Herstellung der neuen Verbindungen IV und VIII kann in an sich bekannter Weise erfolgen. So sind die β-Ketocarbonsäure-(1,4:3,6-dianhydro-hexit)-ester der allgemeinen Formel VIII beispielsweise erhältlich aus den entsprechenden 1,4:3,6-Dianhydro-hexit-Derivaten der allgemeinen Formel IX

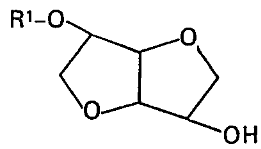

IX

in der $R^1$ die angegebene Bedeutung besitzt, durch Umsetzung mit Diketen oder auf dem Weg der Umesterung mit β-Ketocarbonsäureniederalkylestern (siehe z.B. Houben-Weyl, Methoden der Organischen Chemie, Vol. VII/4, 230 ff [1968)].

Als Beispiele für die Ester der allgemeinen Formel VIII seien genannt:

Acetessigsäure-(5-isosorbid)-ester,
Acetessigsäure-(2-isosorbid)-ester,
Acetessigsäure-isomannid-ester,
Acetessigsäure-isoidid-ester,
Acetessigsäure-(5-isosorbid-2-nitrat)-ester,
Acetessigsäure-(2-isosorbid-5-nitrat)-ester,
Acetessigsäure-(5-isomannid-2-nitrat)-ester,
Acetessigsäure-(5-isoidid-2-nitrat)-ester,
Acetessigsäure-(5-isosorbid-2-acetat)-ester,
Acetessigsäure-(2-isosorbid-5-acetat)-ester,
Acetessigsäure-(5-isomannid-2-acetat)-ester,
Acetessigsäure-(5-isoidid-2-acetat)-ester,
Acetessigsäure-(5-isosorbid-2-butyrat)-ester,
Acetessigsäure-(2-isosorbid-5-butyrat)-ester,
Acetessigsäure-(5-isosorbid-2-nicotinat)-ester,
Acetessigsäure-(2-isosorbid-5-nicotinat)-ester,
Propionylessigsäure-(5-isosorbid-2-nitrat)-ester,
Propionylessigsäure-(2-isosorbid-5-nitrat)-ester,
Propionylessigsäure-(5-isosorbid)-ester,
Propionylessigsäure-(2-isosorbid)-ester,

Propionylessigsäure-(5-isosorbid-2-acetat)-ester,
Propionylessigsäure-(2-isosorbid-5-acetat)-ester,
Propionylessigsäure-isomannid-ester,
Propionylessigsäure-isoidid-ester.

Auch die Enaminocarbonsäure-(1,4:3,6-dianhydro-hexit)-ester der allgemeinen Formel IV können in an sich bekannter Weise hergestellt werden aus den β-Ketocarbonsäure-(1,4:3,6-dianhydro-hexit)-estern der allgemeinen Formel VIII durch Umsetzung mit Ammoniak.

Als Beispiele für die Ester der allgemeinen Formel IV seien genannt:

β-Aminocrotonsäure-(5-isosorbid)-ester,
β-Aminocrotonsäure-(2-isosorbid)-ester,
β-Aminocrotonsäure-isomannid-ester,
β-Aminocrotonsäure-isoidid-ester,
β-Aminocrotonsäure-(5-isosorbid-2-nitrat)-ester,
β-Aminocrotonsäure-(2-isosorbid-5-nitrat)-ester,
β-Aminocrotonsäure-(5-isomannid-2-nitrat)-ester,
β-Aminocrotonsäure-(5-isoidid-2-nitrat)-ester,
β-Aminocrotonsäure-(5-isosorbid-2-acetat)-ester,
β-Aminocrotonsäure-(2-isosorbid-5-acetat)-ester,
β-Aminocrotonsäure-(5-isomannid-2-acetat)-ester,
β-Aminocrotonsäure-(5-isoidid-2-acetat)-ester,
β-Aminocrotonsäure-(5-isosorbid-2-butyrat)-ester,
β-Aminocrotonsäure-(2-isosorbid-5-butyrat)-ester,
β-Aminocrotonsäure-(5-isosorbid-2-nicotinat)-ester,
β-Aminocrotonsäure-(2-isosorbid-5-nicotinat)-ester,
β-Amino-β-ethyl-acrylsäure-(5-isosorbid-2-nitrat)-ester,
β-Amino-β-etyhl-acrylsäure-(2-isosorbid-5-nitrat)-ester,
β-Amino-β-ethyl-acrylsäure-(5-isosorbid)-ester,
β-Amino-β-ethyl-acrylsäure-(2-isosorbid)-ester,
β-Amino-β-ethyl-acrylsäure-(5-isosorbid-2-acetat)-ester,
β-Amin-β-ethyl-acrylsäure-(2-isosorbid-5-acetat)-ester,
β-Amino-β-ethyl-acrylsäure-isomannid-ester,
β-Amino-β-ethyl-acrylsäure-isoidid-ester.

Ferner lassen sich die erfindungsgemässen Verbindungen der allgemeinen Formel I, in welcher $R^2$ die angegebene Bedeutung besitzt und $R^1$ für Wasserstoff steht, nach Variante E dadurch herstellen, dass man Verbindungen der allgemeinen Formel I, worin $R^1$ einen niederen Acylrest mit 2 bis 5 Kohlenstoffatomen oder einen Pyridylcarbonylrest bedeutet und $R^2$ die angegebene Bedeutung besitzt, in an sich bekannter Weise mit einem niederen Alkohol, wie Methanol, Ethanol usw. umestert oder in Gegenwart von Säuren oder Basen einer Hydrolyse unterwirft.

Die Hydrolyse erfolgt vorzugsweise in Gegenwart eines mit Wasser mischbaren organischen Lösungsmittels bei Temperaturen von 0 bis 150°C. Als Lösungsmittel kommen in Frage Alkohole, wie Methanol, Ethanol, Propanol, Isopropanol, ferner Dioxan, Tetrahydrofuran, Eisessig, Dimethylformamid, Dimethylsulfoxid und andere. Als Hydrolyseagentien kommen sowohl Säuren, wie Schwefelsäure, Chlorwasserstoffsäuren und dergleichen, als auch Basen wie Alkalihydroxide, Alkalialkoholate usw. in Betracht.

Die Umesterung mit niederen Alkoholen kann in Gegenwart von Alkalialkoholaten, wie Natrium- oder Kalium-methylat oder -ethylat oder in Gegenwart von basischen Ionenaustauschern, z.B. Dowex, durchgeführt werden. Hierbei arbeitet man vorzugsweise in dem zur Umesterung eingesetzten Alkohol als Lösungsmittel.

Umgekehrt lassen sich Verbindungen der allgemeinen Formel I, in der $R^1$ einen niederen Acylrest mit 2 bis 5 Kohlenstoffatomen oder einen Pyridylcarbonylrest bedeutet und $R^2$ die angegebene Bedeutung besitzt, dadurch herstellen, dass man Verbindungen der allgemeinen Formel I, in der $R^1$ für Wasserstoff steht und $R^2$ die angegebene Bedeutung besitzt, auf an sich bekannte Weise mittels eines entsprechenden Säurechlorids oder -anhydrids acyliert (Variante F).

Aufgrund der beiden möglichen, entgegengesetzten Konfigurationen am C-4-Atom des 1,4-Dihydropyridin-Ringes und der Chiralität der 1,4:3,6-Dianhydro-hexite entstehen bei jeder der Herstellungsvarianten A-D jeweils 2 Diastereoisomere. Diese unterscheiden sich in ihren chemischen und physikalischen Eigenschaften und können daher mit Hilfe bekannter Methoden voneinander getrennt werden. Als Trennverfahren seien beispielsweise genannt: Umkristallisation aus geeigneten, inerten Lösungsmitteln, dünnschicht- oder säulenchromatographische Trennung oder Auftrennung mittels Hochdruckflüssigkeitschromatographie.

Beim Einsatz des Diastereoisomerenpaares in die Herstellungsvarianten E und F erhält man jeweils auch wieder das entsprechende Diastereoisomerenpaar als Reaktionsprodukt. Geht man von den getrennten, konfigurativ einheitlichen Komponenten aus, so erhält man nach E und F einheitliche Komponenten mit der gleichen Konfiguration wie das Ausgangsprodukt.

Sowohl die Diastereoisomerengemische als auch die getrennten, konfigurativ einheitlichen Komponenten sind als pharmazeutische Wirkstoffe verwendbar und gehören zur vorliegenden Erfindung.

Die erfindungsgemässen Acylderivate der 1,4:3,6-Dianhydro-hexite der allgemeinen Formel I und ihre Salze besitzen wertvolle pharmakologische Eigenschaften. Aufgrund ihrer kreislaufbeeinflussenden Wirkung können sie z.B. als antihypertensive Mittel, als periphere und zentrale Vasodilatatoren sowie als Coronartherapeutika Verwendung finden.

Die neuen Wirkstoffe können in bekannter Weise in übliche pharmazeutische Formulierungen übergeführt werden, unter Verwendung geeigneter Träger- und Zusatzstoffe oder Lösungsmittel. Die Applikation erfolgt in üblicher Weise, vorzugsweise oral oder parenteral, d.h. intramuskulär, subkutan, intravenös oder intraperitoneal. Der Träger oder das Verdünnungsmittel wird je nach der gewünschten Applikationsart ausgewählt. Bei oraler Verabreichung können die erfindungsgemässen Arzneimittel beispielsweise in Form von Tabletten, Kapseln, Pastillen, Pulvern, Sirupen, Elixieren, wässrigen Lösungen und Suspensionen und dergleichen angewandt werden in Übereinstimmung mit der üblichen pharmakologischen Praxis. Das Verhältnis zwischen dem Wirkstoff und dem Träger hängt selbstverständlich ab von der chemischen Natur, der Löslichkeit und der Stabilität der Wirkstoffe wie auch von der gewünschten Dosierung. Vorzugsweise enthalten die pharmazeutischen Zusammensetzungen den erfindungsgemässen Wirkstoff in einer Menge von etwa 20 bis 95 Prozent. Im Fall von Tabletten für die orale Verabreichung können als Trägerstoffe die üblicherweise verwendeten Träger wie Laktose, Natriumcitrat und Salze der Phosphorsäure verwendet werden. Zusatzstoffe wie Stärke, Gleitmittel wie Magnesiumstearat, Natriumlaurylsulfat und Talk werden üblicherweise in Tabletten verwendet. Für die orale Verabreichung in Kapselform sind Laktose und hochmolekulare Polyethylenglycole geeignete Verdünnungsmittel. Wenn wässrige Lösungen oder Suspensionen für die orale Verabreichung benötigt werden, kann der Wirkstoff mit Emulgatoren und Suspensionsmitteln kombiniert werden. Gegebenenfalls können bestimmte Süssungsmittel und/oder Geschmackstoffe zugesetzt werden. Für die parenterale Verabreichung werden üblicherweise sterile Lösungen des Wirkstoffes hergestellt und der ph-Wert der Lösungen wird auf geeignete Weise eingestellt und abgepuffert. Für intravenöse Verabreichung soll die Gesamtkonzentration der gelösten Stoffe kontrolliert werden, um das Präparat isotonisch einzustellen.

Obwohl der verabreichende Arzt letztlich die Dosierung bestimmen wird, kann man davon ausgehen, dass die Einzeldosierung der erfindungsgemässen Substanzen bei Mitteln gegen Angina pectoris und gegen Hypertension im allgemeinen im Bereich von 0,10 bis 3,0 mg/kg Körpergewicht, vorzugsweise bei 0,15 bis 1,0 mg/kg Körpergewicht liegt. Für die Anwendung bei Menschen bei der Behandlung von Angina-pectoris-Anfällen können 2 bis 4 sublinguale Tabletten oder Lösungen als Mundspray pro Tag verwendet werden. Für die Behandlung von Hypertension und für die prophylaktische Behandlung der Angina pectoris sollten 2 bis 4 Kapseln, überzogene Pillen, Tabletten oder 1 bis 2 Suppositorien täglich verabreicht werden. Es mag ausreichen in einigen Fällen weniger als die obigen Dosierungen anzuwenden, während in anderen Fällen die oberen Grenzen auch überschritten werden können.

Die nachfolgenden Beispiele dienen der Erläuterung der Erfindung. Die angegebenen Schmelzpunkte sind unkorrigiert. $[\alpha]_D^{20}$ bedeutet die optische Drehung bei 20°C, Natrium-D-Linie. Die Art des Lösungsmittels sowie die Konzentration der gemessenen Lösung in g/100 ml sind in Klammern angefügt. Soweit in den Beispielen Werte für mehr als eine Verbindung gegeben werden, handelt es sich um getrennt isolierte Isomere.

Beispiel 1

5-(1,4-Dihydro-2,6-dimethyl-3-methoxycarbonyl-4-phenyl-5-pyridylcarbonyl)-isosorbid-2-acetat

a) Isosorbid-2-acetat-5-acetoacetat: Zu 376 g Isosorbid-2-acetat und 2 ml Triethylamin tropft man bei 80°C, unter Rühren, 155 ml Diketen und hält das Gemisch noch 1 Std. bei dieser Temperatur. Das entstandene Reaktionsprodukt fällt in öliger Form an und wird ohne weitere Reinigung in die nächste Stufe eingesetzt.

b) Isosorbid-2-acetat-5-(3-amino)-crotonat: Das Rohprodukt aus a) wird in 1 l Ethanol gelöst. Man leitet einen kräftigen Ammoniakstrom bis zur Sättigung ein und lässt danach 2 Std. bei Raumtemperatur stehen. Anschliessend kocht man kurz auf und kühlt im Eisbad ab. Dabei kristallisiert das Reaktionsprodukt aus, das durch Absaugen isoliert wird. Nach dem Umkristallisieren aus Ethanol erhält man 350 g vom Schmp. 123–124°C.

c) 40,8 g Benzyliden-acetessigsäure-methylester und 54,2 g Isosorbid-2-acetat-5-(3-amino)-crotonat werden in 200 ml Ethanol 8 Std. unter Rückfluss erhitzt. Der nach dem Abkühlen erhaltene Niederschlag (42 g) wird abgesaugt und aus Ethanol umkristallisiert. Man erhält so das eine Isomer, (+)-5-(1,4-Dihydro-2,6-dimethyl-3-methoxycarbonyl-4-phenyl-5-pyridylcarbonyl)-isosorbid-2-acetat, Schmp. 189–190°C, $[\alpha]_D^{20}$ +45,37 (c = 1,047, Ethanol).

Aus den Mutterlaugen wird durch Einengen und Umkristallisieren aus Ethanol das andere Isomer, (-)-5-(1,4-Dihydro-2,6-dimethyl-3-methoxycarbonyl-4-phenyl-5-pyridylcarbonyl)-isosorbid-2-acetat, gewonnen. Schmp. 183–184°C, $[\alpha]_D^{20}$ -52,87 (c = 1,012, Ethanol).

Beispiel 2

2-[1,4-Dihydro-2,6-dimethyl-3-(2-methoxyethoxycarbonyl)-4-(3-nitrophenyl)-5-pyridylcarbonyl]-isosorbid-5-nitrat

a) Isosorbid-5-nitrat-2-acetoacetat: Zu 362 g Isosorbid-5-nitrat und 2 ml Triethylamin tropft man bei 80°, unter Rühren, 155 ml Diketen und hält das Gemisch noch 1 Std. bei dieser Temperatur. Das entstandene Reaktionsprodukt fällt in öliger Form an und wird ohne weitere Reinigung in die nächste Stufe eingesetzt.

b) Isosorbid-5-nitrat-2-(3-amino)-crotonat: Das aus a) erhaltene Rohprodukt wird analog Beispiel 1 b mit Ammoniak umgesetzt. Man erhält 375 g Reaktionsprodukt, das nach Umkristallisieren aus Ethanol bei 97,5–98,5°C schmilzt.

c) 15,1 g 3-Nitro-benzaldehyd, 16,0 g Acetessigsäure-(2-methoxy-ethyl)-ester und 27,4 g Isosorbid-5-nitrat-2-(3-amino)-crotonat werden in

300 ml Ethanol 16 Std. unter Rückfluss erhitzt. Der nach dem Abkühlen erhaltene Niederschlag wird abgesaugt und aus Ethanol umkristallisiert. Man erhält 17,6 g (+)-2-[1,4-Dihydro-2,6-dimethyl-3-(2-methoxy-ethoxycarbonyl)-4-(3-nitrophenyl)-5-pyridyl-carbonyl]-isosorbid-5-nitrat, Schmp. 201°C, $[\alpha]_D^{20}$ + 113,2 (c = 0,349, Aceton).

Aus den Mutterlaugen werden durch Einengen und Umkristallisieren aus Isopropanol 11,5 g des anderen Isomers, (+)-2-[1,4-Dihydro-2,6-dimethyl-3-(2-methoxy-ethoxycarbonyl)-4-(3-nitrophenyl)-5-pyridylcarbonyl]-isosorbid-5-nitrat, gewonnen. Schmp. 124°C, $[\alpha]_D^{20}$ + 26,1 (c = 0,556, Aceton).

Beispiel 3

5-[1,4-Dihydro-2,6-dimethyl-3-methoxycarbonyl-4-(3-nitrophenyl)-5-pyridylcarbonyl]-isosorbid-2-nitrat

a) Isosorbid-2-nitrat-5-acetoacetat: Die Herstellung erfolgt analog Beispiel 1 a) aus Isosorbid-2-nitrat und Diketen. Die Substanz wird als öliges Rohprodukt zur weiteren Reaktion eingesetzt.

b) Eine Lösung von 27,5 g Isosorbid-2-nitrat-5-acetoacetat, 15,1 g 3-Nitrobenzaldehyd und 11,5 g 3-Amino-crotonsäure-methylester in 100 ml Ethanol wird 20 Std. unter Rückfluss erhitzt. Danach engt man i. Vak. ein und versetzt den Rückstand mit Ether. Nach einigem Stehen kristallisieren 21 g des schwerer löslichen Isomers aus, das durch Umkristallisieren aus Methanol gereinigt wird. Man erhält (+)-5-[1,4-Dihydro-2,6-dimethyl-3-methoxycarbonyl-4-(3-nitrophenyl)-5-pyridylcarbonyl]-isosorbid-2-nitrat, Schmp. 182,5°C (Zers.), $[\alpha]_D^{20}$ + 33,9 (c = 0,087, Ethanol).

Die etherische Mutterlauge wird eingeengt und der Rückstand durch Umkristallisieren aus Toluol gereinigt. Man erhält das andere Isomer, (–)-5-[1,4-Dihydro-2,6-dimethyl-3-methoxycarbonyl-4-(3-nitrophenyl)-5-pyridyl-carbonyl]-isosorbid-2-nitrat, Schmp. 160°C, $[\alpha]_D^{20}$ – 51,4 (c = 0,292, Ethanol).

Beispiel 4

5-[1,4-Dihydro-2,6-dimethyl-3-methoxycarbonyl-4-(4-cyanophenyl)-5-pyridylcarbonyl]-isosorbid-2-nitrat

In eine Lösung von 27,5 g Isosorbid-2-nitrat-5-acetoacetat (Beispiel 3 a) in 100 ml Ethanol werden 3 g Ammoniak eingeleitet. Danach versetzt man mit 22,9 g (4-Cyano-benzyliden)-acetessigsäure-methylester und erhitzt 20 Std. unter Rückfluss, wobei anfangs noch ein schwacher Ammoniakstrom durch die Lösung geleitet wird. Man engt ein und nimmt den Rückstand in Toluol/Ether 1:1 auf, wobei Kristallisation eintritt. Durch Umkristallisation aus Ethanol wird das eine Isomer erhalten, Schmp. 172°C (Zers.), $[\alpha]_D^{20}$ + 72,1 (c = 0,985, Aceton).

Beispiel 5

5-(1,4-Dihydro-2,6-dimethyl-3-methoxycarbonyl-4-phenyl-5-pyridylcarbonyl)-isosorbid

20 g (+)-5-(1,4-Dihydro-2,6-dimethyl-3-methoxycarbonyl-4-phenyl-5-pyridylcarbonyl)-isosorbid-2-acetat vom Schmp. 189–190°C (Beispiel 1) werden in 100 ml Methanol suspendiert und mit 1 ml 35-proz. methanolischer Natriummethylat-Lösung versetzt. Unter Rühren bei Raumtemperatur entsteht eine klare Lösung, aus der nach einiger Zeit ein Niederschlag ausfällt. Dieser wird abgesaugt und aus Ethanol umkristallisiert. Man erhält 14 g (+)-5-(1,4-Dihydro-2,6-dimethyl-3-methoxycarbonyl-4-phenyl-5-pyridylcarbonyl)-isosorbid, Schmp. 193°C, $[\alpha]_D^{20}$ + 44,2 (c = 1,209, Ethanol).

Beim Einsatz des linksdrehenden Isomers, (–)-5-(1,4-Dihydro-2,6-dimethyl-3-methoxycarbonyl-4-phenyl-5-pyridylcarbonyl)-isosorbid, vom Schmp. 183–184°C (Beispiel 1), erhält man auf analoge Weise das entsprechende Isomer, (–)-5-(1,4-Dihydro-2,6-dimethyl-3-methoxycarbonyl-4-phenyl-5-pyridylcarbonyl)-isosorbid, das mit 0,25 mol Wasser kristallisiert. Schmp. 120–121°C, $[\alpha]_D^{20}$ – 67,5 (c = 1,015, Ethanol).

Beispiel 6

5-[1,4-Dihydro-2,6-dimethyl-3-methoxycarbonyl-4-(3-nitrophenyl)-5-pyridylcarbonyl]-isosorbid

5,0 g (–)-5-[1,4-Dihydro-2,6-dimethyl-3-methoxycarbonyl -4-(3-nitrophenyl)-5-pyridylcarbonyl]-isosorbid-2-acetat (Beispiel 12) und 0,5 g Natriumhydroxid werden in 20 ml Methanol gelöst und einige Zeit bei Raumtemperatur gerührt. Der entstandene Niederschlag wird abgesaugt und umkristallisiert. Man erhält 3,9 g (–)-5-[1,4-Dihydro-2,6-dimethyl-3-methoxycarbonyl-4-(3-nitrophenyl)-5-pyridylcarbonyl]-isosorbid. Die Substanz kristallisiert mit 1 mol Methanol und zeigt in dieser Form einen Schmp. von 163°C, $[\alpha]_D^{20}$ – 37,6 (c = 1,077, Ethanol).

Auf gleiche Weise erhält man aus (+)-5-[1,4-Dihydro-2,6-dimethyl-3-methoxycarbonyl-4-(3-nitrophenyl)-5-pyridylcarbonyl]-isosorbid-2-acetat (Beispiel 12) das entsprechende Isomer, Schmp. 155°C, $[\alpha]_D^{20}$ + 53,64 (c = 0,811, Ethanol).

Beispiel 7

5-[1,4-Dihydro-2,6-dimethyl-3-methoxycarbonyl-4-(2-nitro-phenyl)-5-pyridylcarbonyl]-isosorbid-2-butyrat

10 g (+)-5-[1,4-Dihydro-2,6-dimethyl-3-methoxycarbonyl-4-(2-nitrophenyl)-5-pyridylcarbonyl]-isosorbid (Beispiel 24) werden mit 10 g Buttersäure-anhydrid und 0,1 g 4-Dimethyl-amino-pyridin 30 Min. bei 60°C gerührt. Beim Verdünnen des Reaktionsgemisches mit 100 ml Ether erhält man eine kristalline Fällung, die abgesaugt und aus Ethanol umkristallisiert wird. Ausbeute: 9,8 g, Schmp. 161–163°C, $[\alpha]_D^{20}$ + 182,06 (c = 1,126, Aceton).

Beim Einsatz des linksdrehenden Isomers (Beispiel 24) wird das entsprechende Isomer erhalten. Schmp. 153–156°C (aus Toluol/Ether), $[\alpha]_D^{20}$ – 195,53 (c = 1,097, Aceton).

Die nachfolgenden Verbindungen werden nach einer der in den Beispielen 1 bis 7 angegebenen Methoden erhalten.

Beispiel 8

5-[1,4-Dihydro-2,6-dimethyl-3-methoxycarbonyl-4-(2-nitrophenyl)-5-pyridylcarbonyl]-isosorbid-2-nitrat

a) Aus Isosorbid-2-nitrat-5-acetoacetat (Beispiel 3) erhält man Isosorbid-2-nitrat-5-(3-amino)-crotonat, Schmp. 118°C (aus Ethanol).

b) Schmp. 217–219°C (Zers.) (aus Acetonitril), $[\alpha]_D^{20}$ + 195,8 (c = 0,036, Ethanol).

Schmp. 79–81°C (aus Acetonitril), $[\alpha]_D^{20}$ – 268,0 (c = 0,25, Ethanol).

Beispiel 9

2-[1,4-Dihydro-2,6-dimethyl-3-methoxycarbonyl-4-(2-nitrophenyl)-5-pyridylcarbonyl]-isosorbid-5-nitrat

Schmp. 207°C (Zers.) (aus Acetonitril), $[\alpha]_D^{20}$ – 168,2 (c = 0,217, Ethanol).

Schmp. 186–187°C (aus Ethanol), $[\alpha]_D^{20}$ + 162,6 (c = 0,376, Ethanol).

Beispiel 10

5-(1,4-Dihydro-2,6-dimethyl-3-methoxycarbonyl-4-phenyl-5-pyridylcarbonyl)-isosorbid-2-nitrat

Schmp. 188–189°C (Zers.) (aus Ethanol), $[\alpha]_D^{20}$ + 11,7 (c = 0,256, Ethanol).

Beispiel 11

2-(1,4-Dihydro-2,6-dimethyl-3-methoxycarbonyl-4-phenyl-5-pyridylcarbonyl)-isosorbid-5-nitrat

Schmp. 147–148°C (aus Toluol), $[\alpha]_D^{20}$ + 115,1 (c = 0,364, Ethanol).

Schmp. 146°C (aus Toluol), $[\alpha]_D^{20}$ + 66,96 (c = 1,064, Ethanol).

Beispiel 12

5-[1,4-Dihydro-2,6-dimethyl-3-methoxycarbonyl-4-(3-nitrophenyl)-5-pyridylcarbonyl]-isosorbid-2-acetat

Schmp. 216–217°C (aus Ethanol), $[\alpha]_D^{20}$ + 48,39 (c = 0,248, Ethanol).

Schmp. 94–97°C (aus Ether), $[\alpha]_D^{20}$ – 16,6 (c = 0,965, Ethanol).

Beispiel 13

5-[1,4-Dihydro-2,6-dimethyl-3-methoxycarbonyl-4-(2-nitrophenyl)-5-pyridylcarbonyl]-isosorbid-2-acetat

Schmp. 212°C (aus Acetonitril), $[\alpha]_D^{20}$ + 48,74 (c = 0,658, Aceton).

Schmp. 205–207°C (aus Ethanol), $[\alpha]_D^{20}$ –157,8 (c = 0,952, Aceton).

Beispiel 14

2-[1,4-Dihydro-2,6-dimethyl-3-methoxycarbonyl-4-(3-nitrophenyl)-5-pyridylcarbonyl]-isosorbid-5-nitrat

Isomerengemisch: Schmp. 204°C (Zers.) (aus Ethanol), $[\alpha]_D^{20}$ + 74,0 (c = 1,051, Aceton).

Beispiel 15

2-[1,4-Dihydro-2,6-dimethyl-3-methoxycarbonyl-4-(3-nitrophenyl)-5-pyridylcarbonyl]-isosorbid

a) Isosorbid-2-(3-amino)-crotonat: Schmp. 140°C (aus Ethanol).

b) Schmp. 215°C (aus Ethanol), $[\alpha]_D^{20}$ + 83,25 (c = 1,003, Aceton).

Beispiel 16

2-[1,4-Dihydro-2,6-dimethyl-3-butoxycarbonyl-4-(3-nitrophenyl)-5-pyridylcarbonyl]-isosorbid-5-nitrat

Schmp. 193–194°C (aus Ethanol), $[\alpha]_D^{20}$ + 104,8 (c = 0,501, Aceton).

Schmp. 170–171°C (aus Methanol), $[\alpha]_D^{20}$ + 34,6 (c = 0,224, Aceton).

Beispiel 17

2-[1,4-Dihydro-2,6-dimethyl-3-isopropoxycarbonyl-4-(3-nitrophenyl)-5-pyridylcarbonyl]-isosorbid-5-nitrat

Isomerengemisch: Schmp. 183–184°C (Zers.) (aus Isopropanol), $[\alpha]_D^{20}$ + 119,5 (c = 0,544, Aceton).

Beispiel 18

2-[1,4-Dihydro-2,6-dimethyl-3-ethoxycarbonyl-4-(3-nitrophenyl)-5-pyridylcarbonyl]-isosorbid-5-nitrat

Isomerengemisch: Schmp. 161°C (aus Ethanol), $[\alpha]_D^{20}$ + 60,5 (c = 0,991, Aceton).

Beispiel 19

2-[1,4-Dihydro-2,6-dimethyl-3-allyloxycarbonyl-4-(3-nitrophenyl)-5-pyridylcarbonyl]-isosorbid-5-nitrat

Isomerengemisch: Schmp. 144°C (aus Ethanol), $[\alpha]_D^{20}$ + 70,8 (c = 1,024, Aceton).

Beispiel 20

2-[1,4-Dihydro-2,6-dimethyl-3-isobutoxycarbonyl-4-(3-nitrophenyl)-5-pyridylcarbonyl]-isosorbid-5-nitrat

Isomerengemisch: Schmp. 173°C (Zers.) (aus Ethanol), $[\alpha]_D^{20}$ + 62,0 (c = 1,016, Aceton).

Beispiel 21

2-[1,4-Dihydro-2,6-dimethyl-3-(tert.-butoxycarbonyl)-4-(3-nitrophenyl)-5-pyridylcarbonyl]-isosorbid-5-nitrat

Schmp. 192°C (Zers.) (aus Methanol), $[\alpha]_D^{20}$ + 24,6 (c = 1,035, Aceton).

Kristallisiert mit 0,25 mol Wasser: Schmp. 122–123°C (aus Isopropanol), $[\alpha]_D^{20}$ + 110,2 (c = 0,935, Aceton).

Beispiel 22

2-[1,4-Dihydro-2,6-dimethyl-3-methoxycarbonyl-4-(2-nitrophenyl)-5-pyridylcarbonyl]-isosorbid

Schmp. 192–194°C (aus Ethanol), $[\alpha]_D^{20}$ + 290,0 (c = 1,006, Aceton).

Kristallisiert mit 0,25 mol Wasser: Schmp. 172–177°C (aus Ethanol), $[\alpha]_D^{20}$ −228,6 (c = 1,111, Ethanol).

Beispiel 23

2-[1,4-Dihydro-2,6-dimethyl-3-methoxycarbonyl-4-(2-nitrophenyl)-5-pyridylcarbonyl]-isosorbid-5-acetat

a) Isosorbid-5-acetat-2-(3-amino)-crotonat: Die Herstellung erfolgt aus Isosorbid-5-acetat, über das ölige Isosorbid-5-acetat-2-acetoacetat, wie in Beispiel 1 beschrieben. Die Substanz stellt ein viscoses Öl dar, das in dieser Form für die weitere Umsetzung eingesetzt wird.

b) Schmp. 198–200°C (aus Ethanol), $[\alpha]_D^{20}$ + 304,2 (c = 1,005, Aceton).

Schmp. 146–149°C (aus Ethanol), $[\alpha]_D^{20}$ − 168,7 (c = 0,990, Ethanol).

Beispiel 24

5-[1,4-Dihydro-2,6-dimethyl-3-methoxycarbonyl-4-(2-nitrophenyl)-5-pyridylcarbonyl]-isosorbid

a) Isosorbid-5-(3-amino)-crotonat: Schmp. 138°C (aus Ethanol)

b) Schmp. 235°C (aus Acetonitril), $[\alpha]_D^{20}$ + 209,9 (c = 1,017, Aceton).

Schmp. 188–189°C (aus Ethanol), $[\alpha]_D^{20}$ − 228,1 (1,083, Aceton).

Beispiel 25

2-[1,4-Dihydro-2,6-dimethyl-3-methoxycarbonyl-4-(3-nitrophenyl)-5-pyridylcarbonyl]-isosorbid-5-acetat

Schmp. 131–134°C (aus Toluol/Ether), $[\alpha]_D^{20}$ + 108,5 (c = 0,968, Ethanol).

Beispiel 26

2-[1,4-Dihydro-2,6-dimethyl-3-methoxycarbonyl-4-(3,4-methylendioxy-phenyl)-5-pyridylcarbonyl]-isosorbid-5-nitrat

Schmp. 189°C (aus Toluol), $[\alpha]_D^{20}$ + 124,6 (c = 1,011, Aceton). Schmp. 154–156°C (aus Ethanol/Ether), $[\alpha]_D^{20}$ + 41,8 (c = 0,98, Aceton).

Beispiel 27

5-[1,4-Dihydro-2,6-dimethyl-3-methoxycarbonyl-4-(4-fluorphenyl)-5-pyridylcarbonyl]-isosorbid-2-nitrat

Schmp. 167°C (aus Ethanol), $[\alpha]_D^{20}$ + 30,7 (c = 0,936, Ethanol).

Beispiel 28

2-[1,4-Dihydro-2,6-dimethyl-3-methoxycarbonyl-4-(4-fluorphenyl)-5-pyridylcarbonyl]-isosorbid-5-nitrat

Kristallisiert mit 1 mol Ethanol: Schmp. 108–10°C (aus Ethanol), $[\alpha]_D^{20}$ + 114,6 (c = 1,065, Aceton).

Beispiel 29

2-[1,4-Dihydro-2,6-dimethyl-3-methoxycarbonyl-4-(2-methoxyphenyl)-5-pyridylcarbonyl]-isosorbid-5-nitrat

Schmp. 169°C (aus Ethanol), $[\alpha]_D^{20}$ + 136,7 (c = 1,035, Aceton).

Beispiel 30

2-[1,4-Dihydro-2,6-dimethyl-3-methoxycarbonyl-4-(4-cyanophenyl)-5-pyridylcarbonyl]-isosorbid-5-nitrat

Schmp. 140°C (Zers.) (aus Methanol), $[\alpha]_D^{20}$ + 134,4 (c = 1,019, Aceton).

Schmp. 110°C (aus Methanol), $[\alpha]_D^{20}$ + 22,1 (c = 0,997, Aceton).

Beispiel 31

2-[1,4-Dihydro-2,6-dimethyl-3-methoxycarbonyl-4-(3-trifluormethyl-phenyl)-5-pyridylcarbonyl]-isosorbid-5-nitrat

Isomerengemisch: Schmp. 171–172°C (aus Methanol), $[\alpha]_D^{20}$ + 73,9 (c = 1,055, Aceton).

Beispiel 32

5-[1,4-Dihydro-2,6-dimethyl-3-methoxycarbonyl-4-(3-trifluormethyl-phenyl)-5-pyridylcarbonyl]-isosorbid-2-nitrat

Schmp. 178–179°C (aus Methanol), $[\alpha]_D^{20}$ + 56,0 (c = 1,00, Aceton).

Beispiel 33

2-[1,4-Dihydro-2,6-dimethyl-3-(3-ethoxy-propoxycarbonyl)-4-(3-nitrophenyl)-5-pyridylcarbonyl]-isosorbid-5-nitrat

kristallisiert mit 0,5 mol Wasser: Schmp. 127°C (aus Ethanol), $[\alpha]_D^{20}$ + 37,7 (c = 1,100, Aceton).

Beispiel 34

5-[1,4-Dihydro-2,6-dimethyl-3-methoxycarbonyl-4-(3,4-methylendioxy-phenyl)-5-pyridylcarbonyl]-isosorbid-2-nitrat

Schmp. 169–171°C (aus Ethanol), $[\alpha]_D^{20}$ + 55,7 (c = 1,014, Aceton).

Beispiel 35

2-[1,4-Dihydro-3-ethoxycarbonyl-2-ethyl-6-methyl-4-(3-nitrophenyl)-5-pyridylcarbonyl]-isosorbid-5-nitrat

Schmp. 147°C (aus Ethanol), $[\alpha]_D^{20}$ + 24,42 (c = 0,819, Aceton).

Beispiel 36

5-[1,4-Dihydro-2,6-dimethyl-3-methoxycarbonyl-4-(3-nitrophenyl)-5-pyridylcarbonyl]-isomannid-2-acetat

a) Isomannid-2-acetat-5-acetoacetat: Die Herstellung erfolgt analog Beispiel 1 a) aus Isomannid-2-acetat und Diketen. Die Substanz wird als öliges Rohprodukt zur weiteren Reaktion eingesetzt.

b) Isomannid-2-acetat-5-(3-amino)-crotonat: Das aus a) erhaltene Rohprodukt wird analog Beispiel 1 b) mit Ammoniak umgesetzt. Schmp. 120°C (aus Ethanol).

c) Isomerengemisch: Schmp. 184–185°C (aus Methanol), $[\alpha]_D^{20}$ + 118 (c = 1,018, Aceton).

Beispiel 37

5-[1,4-Dihydro-2,6-dimethyl-3-methoxycarbonyl-4-(3-nitrophenyl)-5-pyridylcarbonyl]-isomannid

Isomerengemisch: Schmp. 141°C (aus Isopropanol), $[\alpha]_D^{20}$ + 90,4 (c = 1,001, Aceton).

Beispiel 38

5-[1,4-Dihydro-2,6-dimethyl-3-methoxycarbonyl-4-(3-nitrophenyl)-5-pyridylcarbonyl]-isomannid-2-nitrat

a) Isomannid-5-acetoacetat-2-nitrat: Die Herstellung erfolgt analog Beispiel 1 a) aus Isomannid-2-nitrat und Diketen. Die Substanz wird als öliges Rohprodukt zur weiteren Reaktion eingesetzt.

b) Isomannid-5-(3-amino)-crotonat-2-nitrat: Das aus a) erhaltene Rohprodukt wird analog Beispiel 1 b) mit Ammoniak umgesetzt. Schmp. 95–96°C (aus Isopropanol).

c) Schmp. 176°C (aus Ethanol), $[\alpha]_D^{20}$ + 95,4 (c = 1,074, Aceton).

Schmp. 153°C (aus Methanol), $[\alpha]_D^{20}$ + 176,2 (c = 1,106, Aceton).

Beispiel 39

5-[1,4-Dihydro-2,6-dimethyl-3-methoxycarbonyl-4-(3-nitrophenyl)-5-pyridylcarbonyl]-isoidid-2-acetat

a) Isoidid-2-acetat-5-acetoacetat: Die Herstellung erfolgt analog Beispiel 1 a) aus Isoidid-2-acetat und Diketen. Die Substanz wird als öliges Rohprodukt zur weiteren Reaktion eingesetzt.

b) Isoidid-2-acetat-5-(3-amino)-crotonat: Das aus a) erhaltene Rohprodukt wird analog Beispiel 1 b) mit Ammoniak umgesetzt. Schmp. 115–116°C (aus Ethanol).

c) Isomerengemisch: Schmp. 150°C (ab 80°C sintern) (aus Ethylacetat/Petrolether), $[\alpha]_D^{20}$ + 154,4 (c = 1,017, Aceton)

Beispiel 40

5-[1,4-Dihydro-2,6-dimethyl-3-methoxycarbonyl-4-(3-nitrophenyl)-5-pyridylcarbonyl]-isoidid

Isomerengemisch: Schmp. 150°C (ab 80°C sintern) aus Ethylacetat/Petrolether), $[\alpha]_D^{20}$ + 46,4 (c = 0,991, Aceton)

Beispiel 41

5-[1,4-Dihydro-2,6-dimethyl-3-methoxycarbonyl-4-(3-nitrophenyl)-5-pyridylcarbonyl]-isosorbid-2-butyrat

Schmp. 109–111°C (aus Ether), $[\alpha]_D^{20}$ + 54,26 (c = 1,069, Aceton).

Beispiel 42

5-[1,4-Dihydro-2,6-dimethyl-3-methoxycarbonyl-4-(3-nitrophenyl)-5-pyridylcarbonyl]-isosorbid-2-nicotinat

Kristallisiert mit 0,5 mol Wasser: Schmp. 224–226°C (aus Dichlormethan/Ether), $[\alpha]_D^{20}$ + 21,77 (c = 0,643, Aceton).

Schmp. 215°C (aus Methanol), $[\alpha]_D^{20}$ – 22,9 (c = 1,109, Aceton).

Beispiel 43

5-[1,4-Dihydro-2,6-dimethyl-3-methoxycarbonyl-4-(2-chlorphenyl)-5-pyridylcarbonyl]-isosorbid-2-nitrat

Schmp. 193°C (aus Methanol), $[\alpha]_D^{20}$ + 130,0 (c = 0,796, Aceton).

Beispiel 44

5-[1,4-Dihydro-2,6-dimethyl-3-methoxycarbonyl-4-(2-methylphenyl)-5-pyridylcarbonyl]-isosorbid-2-nitrat

Schmp. 194–195°C (Zers.) (aus Methanol), $[\alpha]_D^{20}$ + 146,0 (c = 1,011, Aceton).

Beispiel 45

2-[1,4-Dihydro-2,6-dimethyl-3-methoxycarbonyl-4-(2-methylphenyl)-5-pyridylcarbonyl]-isosorbid-5-nitrat

Schmp. 193°C (Zers.) (aus Methanol), $[\alpha]_D^{20}$ + 242,7 (c = 1,026, Aceton).

Beispiel 46

2-[1,4-Dihydro-2,6-dimethyl-3-methoxycarbonyl-4-(3-bromphenyl)-5-pyridylcarbonyl]-isosorbid-5-nitrat

Isomerengemisch: Schmp. 170°C (aus Methanol), $[\alpha]_D^{20}$ + 232,8 (c = 0,638, Aceton).

Beispiel 47

5-[1,4-Dihydro-2,6-dimethyl-3-methoxycarbonyl)-4-(3-bromphenyl)-5-pyridylcarbonyl]-isosorbid-2-nitrat

Schmp. 190°C (aus Ethanol), $[\alpha]_D^{20}$ + 147,1 (c = 0,904, Aceton).

Beispiel 48

2-[1,4-Dihydro-2,6-dimethyl-3-(2-cyanoethoxy)-carbonyl-4-(3-nitrophenyl)-5-pyridylcarbonyl]-isosorbid-5-nitrat

Schmp. 208°C (Zers.) (aus Acetonitril), $[\alpha]_D^{20}$ + 155,4 (c = 1,094, Aceton).

Beispiel 49

5-[1,4-Dihydro-2,6-dimethyl-3-methoxycarbonyl-4-(3-nitrophenyl)-5-pyridylcarbonyl]-isoidid-2-nitrat

a) Isoidid-5-acetoacetat-2-nitrat: Die Herstellung erfolgt analog Beispiel 1 a) aus Isoidid-2-nitrat und Diketen. Die Substanz wird als öliges Rohprodukt zur weiteren Reaktion eingesetzt.

b) Isoidid-5-(3-amino)-crotonat-2-nitrat: Das aus a) erhaltene Rohprodukt wird analog Beispiel 1 b) mit Ammoniak umgesetzt und das ölige Reaktionsprodukt über eine mit Kieselgel gefüllte Säule gereinigt.

c) Schmp. 149°C (aus Diethylether), $[\alpha]_D^{20}$ + 8,93 (c = 0,504, Aceton).

Schmp. 141–142°C (aus Diethylether), $[\alpha]_D^{20}$ + 115,66 (c = 0,582, Aceton).

Beispiel 50

5-[1,4-Dihydro-2,6-dimethyl-3-methoxycarbonyl-4-(4-dimethylamino-phenyl)-5-pyridylcarbonyl]-isosorbid-2-nitrat

Schmp. 138°C (aus Methanol), $[\alpha]_D^{20}$ + 60,2 (c = 0,947, Aceton).

Beispiel 51

2-[1,4-Dihydro-2,6-dimethyl-3-methoxycarbonyl-4-(2-chlorphenyl)-5-pyridylcarbonyl]-isosorbid-5-nitrat

Isomerengemisch: Schmp. 154–157°C (aus Methanol/Wasser), $[\alpha]_D^{20}$ + 159,7 (c = 1,021, Aceton).

Beispiel 52

5-[1,4-Dihydro-2,6-dimethyl-3-methoxycarbonyl-4-(2,4-dichlorphenyl)-5-pyridylcarbonyl]-isosorbid-2-nitrat

Schmp. 199–202°C (Zers.) (aus Methanol), $[\alpha]_D^{20}$ + 131,9 (c = 0,959, Aceton).

Beispiel 53

2-[1,4-Dihydro-2,6-dimethyl-3-methoxycarbonyl-4-(3,4,5-trimethoxyphenyl)-5-pyridylcarbonyl]-isosorbid-5-nitrat

Schmp. 187–188°C (aus Methanol), $[\alpha]_D^{20}$ + 127,14 (c = 0,488, Aceton).

Beispiel 54

2-[1,4-Dihydro-2,6-dimethyl-3-methoxycarbonyl-4-(4-chlor-3-nitro-phenyl)-5-pyridylcarbonyl]-isosorbid-2-nitrat

Schmp. 150°C (aus Ethanol), $[\alpha]_D^{20}$ + 41,3 (c = 0,878, Aceton).

Beispiel 55

2-[1,4-Dihydro-2,6-dimethyl-3-methoxycarbonyl-4-(4-chlor-3-nitro-phenyl)-5-pyridylcarbonyl]-isosorbid-5-nitrat

Schmp. 182°C (aus Methanol), $[\alpha]_D^{20}$ + 100,18 (c = 0,544, Aceton).

Schmp. 154°C (aus Methanol), $[\alpha]_D^{20}$ + 12,25 (c = 0,408, Aceton).

Beispiel 56

5-[1,4-Dihydro-2,6-dimethyl-3-(2-methoxyethoxycarbonyl)-4-(3-nitrophenyl)-5-pyridylcarbonyl]-isosorbid

Schmp. 213°C (aus Ethanol), $[\alpha]_D^{20}$ – 32,1 (c = 0,862, Aceton).

Beispiel 57

5-[1,4-Dihydro-2,6-dimethyl-3-(isopropoxy-carbonyl)-4-(3-nitrophenyl)-5-pyridylcarbonyl]-isosorbid

Schmp. 152–153°C (aus Isopropanol/Diisopropylether), $[\alpha]_D^{20}$ + 59,2 (c = 0,972, Aceton).

Beispiel 58

5-[1,4-Dihydro-2,6-dimethyl-3-ethoxycarbonyl-4-(3-nitrophenyl)-5-pyridylcarbonyl]-isosorbid

Schmp. 164°C (aus Ethanol), $[\alpha]_D^{20}$ + 55,72 (c = 1,041, Aceton).

Beispiel 59

5-[1,4-Dihydro-2,6-dimethyl-3-(2-cyanoethoxy)-carbonyl-4-(3-nitrophenyl)-5-pyridylcarbonyl]-isosorbid-2-acetat

Schmp. 194°C (aus Ethylacetat), $[\alpha]_D^{20}$ – 8,15 (c = 0,982, Aceton).

Schmp. 184°C (aus Ethanol), $[\alpha]_D^{20}$ + 31,75 (c = 0,992, Aceton).

Beispiel 60

5-[1,4-Dihydro-2,6-dimethyl-3-(2-cyanoethoxy)-carbonyl-4-(3-nitrophenyl)-5-pyridylcarbonyl]-isosorbid

Schmp. 241°C (aus Acetonitril), $[\alpha]_D^{20}$ – 11,96 (c = 1,003, Aceton).

Schmp. 181°C (aus Ethylacetat), $[\alpha]_D^{20}$ + 6,97 (c = 1,076, Aceton).

**Patentansprüche**

1. Acylderivate von 1,4:3,6-Dianhydro-hexiten der allgemeinen Formel I

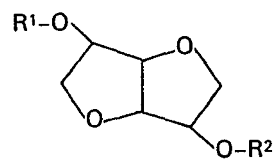

I

in welcher

$R^1$ für Wasserstoff, einen niederen Acylrest mit 2 bis 5 Kohlenstoffatomen, einen Pyridylcarbonylrest oder $NO_2$ steht und

$R^2$ für einen 1,4-Dihydropyridyl-carbonyl-Rest der allgemeinen Formel II steht

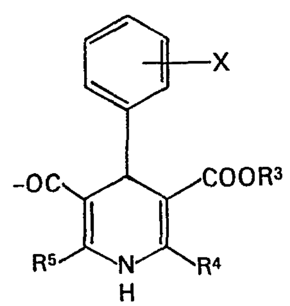

II

in welcher

X für Wasserstoff oder 1,2 oder 3 gleiche oder verschiedene Substituenten aus der Gruppe $C_{1-5}$-Alkoxy, $C_{1-5}$-Alkyl, Cyano, Di-$C_{1-5}$-alkylamino, Halogen, Nitro oder Trifluormethyl oder für eine Methylendioxy-Gruppe steht,

$R^3$ einen geradkettigen oder verzweigten, gesättigten oder ungesättigten Kohlenwasserstoffrest mit 1 bis 5 Kohlenstoffatomen, wobei gegebenenfalls die Kette durch ein Sauerstoffatom unterbrochen und/oder der Kohlenwasserstoffrest gegebenenfalls durch eine Cyanogruppe substituiert sein kann, bedeutet,

$R^4$ und $R^5$ gleich oder verschieden sind und jeweils für eine $C_{1-5}$-Alkylgruppe stehen, sowie pharmazeutisch akzeptable Salze von zur Salzbildung befähigten Verbindungen.

2. Acylderivate gemäss Anspruch 1, dadurch gekennzeichnet, dass sie Derivate des 1,4:3,6-Dianhydro-L-idits (Isoidid) der allgemeinen Formel I a sind

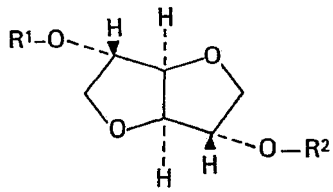

I a

in welcher $R^1$ und $R^2$ die in Anspruch 1 angegebene Bedeutung besitzen.

3. Acylderivate gemäss Anspruch 1, dadurch gekennzeichnet, dass sie Derivate des 1,4:3,6-Dianhydro-D-glucits (Isosorbid) der allgemeinen Formeln I b und I c sind

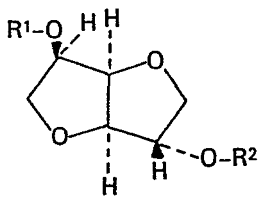

I b

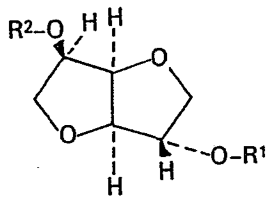

I c

in welcher $R^1$ und $R^2$ die in Anspruch 1 angegebene Bedeutung besitzen.

4. Acylderivate gemäss Anspruch 1, dadurch gekennzeichnet, dass sie Derivate des 1,4:3,6-Dianhydro-D-mannits (Isomannid) der allgemeinen Formel I d sind

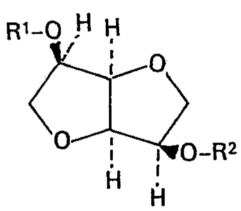

I d

in welcher $R^1$ und $R^2$ die in Anspruch 1 angegebene Bedeutung besitzen.

5. 5-[1,4-Dihydro-2,6-dimethyl-3-methoxycarbonyl-4-(3-nitrophenyl)-5-pyridylcarbonyl]-isosorbid

6. 5-[1,4-Dihydro-2,6-dimethyl-3-methoxycarbonyl-4-(2-nitrophenyl)-5-pyridylcarbonyl]-isosorbid

7. Verfahren zur Herstellung der Acylderivate gemäss Anspruch 1, dadurch gekennzeichnet, dass man

A) einen Aryliden-β-ketocarbonsäure-ester der allgemeinen Formel III

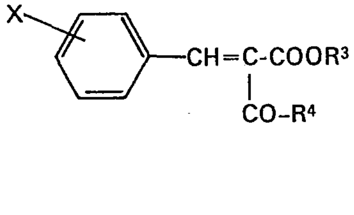

III

in der X, $R^3$ und $R^4$ die in Anspruch 1 angegebene Bedeutung besitzen, in an sich bekannter Weise, mit einem Enaminocarbonsäure-(1,4:3,6-dianhydro-hexit)-ester der allgemeinen Formel IV

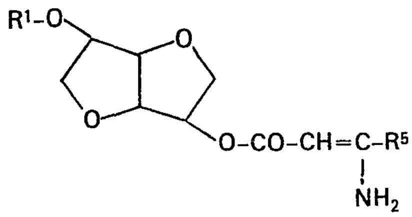

IV

in der $R^1$ und $R^5$ die in Anspruch 1 angegebene Bedeutung besitzen, umsetzt oder

B) einen Aldehyd der allgemeinen Formel V

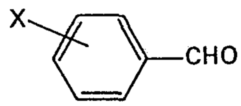

V

in der X die in Anspruch 1 angegebene Bedeutung besitzt, in an sich bekannter Weise mit einem β-Ketocarbonsäure-ester der allgemeinen Formel VI

$R^4-CO-CH_2-COOR^3$ VI

in der $R^3$ und $R^4$ die in Anspruch 1 angegebene Bedeutung besitzen und mit einem Enaminocarbonsäure-(1,4:3,6-dianhydro-hexit)-ester der allgemeinen Formel IV, in der $R^1$ und $R^5$ die in Anspruch 1 angegebene Bedeutung besitzen, umsetzt oder

C) einen Aldehyd der allgemeinen Formel V in der X die in Anspruch 1 angegebene Bedeutung besitzt, in an sich bekannter Weise, mit einem Enaminocarbonsäureester der allgemeinen Formel VII

$R^4-C=CH-COOR^3$ VII
$\;\;|$
$\;NH_2$

in der $R^3$ und $R^4$ die in Anspruch 1 angegebene Bedeutung besitzen und mit einem β-Ketocarbonsäure-(1,4:3,6-dianhydro-hexit)-ester der allgemeinen Formel VIII

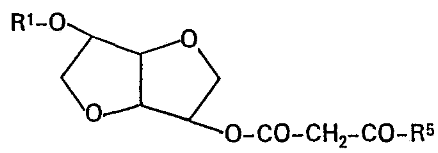

VIII

in der $R^1$ und $R^5$ die in Anspruch 1 angegebene Bedeutung besitzen, umsetzt, oder

D) einen Aryliden-β-ketocarbonsäure-ester der allgemeinen Formel III, in der X, $R^3$ und $R^4$ die in Anspruch 1 angegebene Bedeutung besitzen, in an sich bekannter Weise mit einem β-Ketocarbonsäure-(1,4:3,6-dianhydro-hexit)-ester der allgemeinen Formel VIII, in der $R^1$ und $R^5$ die in An-

spruch 1 angegebene Bedeutung besitzen und mit Ammoniak umsetzt,

und gegebenenfalls ein so erhaltenes Acylderivat der allgemeinen Formel I in an sich bekannter Weise in ein Salz überführt.

8. Verfahren zur Herstellung von Acylderivaten gemäss Anspruch 1, wobei in der allgemeinen Formel I $R^1$ Wasserstoff bedeutet, und $R^2$ die in Anspruch 1 angegebene Bedeutung besitzt, dadurch gekennzeichnet, dass man Verbindungen der allgemeinen Formel I, worin $R^1$ einen niederen Acylrest mit 2 bis 5 Kohlenstoffatomen oder einen Pyridylcarbonylrest bedeutet, und $R^2$ die in Anspruch 1 angegebene Bedeutung besitzt, in an sich bekannter Weise mit einem niederen Alkohol umestert oder in Gegenwart von Säuren oder Basen einer Hydrolyse unterwirft, und gegebenenfalls ein so erhaltenes Acylderivat der allgemeinen Formel I in an sich bekannter Weise in ein Salz überführt.

9. Verfahren zur Herstellung von Acylderivaten gemäss Anspruch 1, wobei in der allgemeinen Formel I $R^1$ einen niederen Acylrest mit 2 bis 5 Kohlenstoffatomen oder einen Pyridylcarbonylrest bedeutet und $R^2$ die in Anspruch 1 angegebene Bedeutung besitzt, dadurch gekennzeichnet, dass man Verbindungen der allgemeinen Formel I, worin $R^1$ Wasserstoff bedeutet und $R^2$ die in Anspruch 1 angegebene Bedeutung besitzt, auf an sich bekannte Weise, mittels eines entsprechenden Säurechlorids oder -anhydrids, acyliert, und gegebenenfalls ein so erhaltenes Acylderivat der allgemeinen Formel I in an sich bekannter Weise in ein Salz überführt.

10. Pharmazeutische Zubereitungen gekennzeichnet durch einen Gehalt an mindestens einer Verbindung gemäss einem der Ansprüche 1 bis 6 sowie üblichen Trägern und/oder Zusatzstoffen.

11. Pharmazeutische Zubereitungen zur Bekämpfung kreislaufbedingter Erkrankungen, gekennzeichnet durch einen Gehalt an mindestens einer Verbindung gemäss einem der Ansprüche 1 bis 6 sowie üblichen Trägern und/oder Zusatzstoffen.

12. Acylderivate gemäss Anspruch 3, dadurch gekennzeichnet, dass sie die allgemeine Formel Ib in Anspruch 3 aufweisen, worin $R^3$, $R^4$ und $R^5$ jeweils Methyl bedeuten.

**Claims**

1. Acyl derivatives of 1,4:3,6-dianhydro-hexites of the general formula I

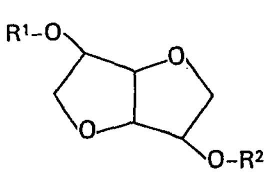

I

in which

$R^1$ stands for hydrogen, a lower acyl radical with 2 to 5 carbon atoms, a pyridylcarbonyl radical or $NO_2$ and

$R^2$ stands for a 1,4-dihydropyridyl-carbonyl radical of the general formula II

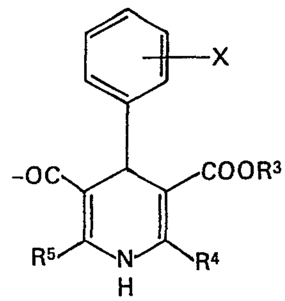

II

in which

X stands for hydrogen or 1,2 or 3 identical or different substituents from the group $C_{1-5}$-alkoxy, $C_{1-5}$-alkyl, cyano, di-$C_{1-5}$-alkylamino, halogen, nitro or trifluoromethyl or for a methylene-dioxy group

$R^3$ stands for a straight-chained or branched, saturated or unsaturated hydrocarbon radical with 1 to 5 carbon atoms, whereby optionally the chain may be interrupted by an oxygen atom and/or the hydrocarbon radical may optionally be substituted by a cyano group,

$R^4$ and $R^5$ are identical or different and each stand for a $C_{1-5}$ alkyl group,

as well as pharmaceutically acceptable salts of compounds capable of salt formation.

2. Acyl derivatives according to claim 1, characterized in that they are derivatives of 1,4:3,6-dianhydro-L-idite (isoidide) of the general formula I a

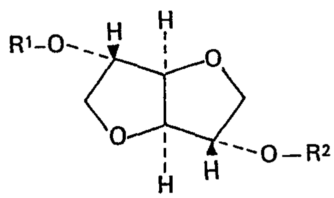

I a

in which $R^1$ and $R^2$ have the meaning as given in claim 1.

3. Acyl derivatives according to claim 1, characterized in that they are derivatives of 1,4:3,6-dianhydro-D-glucite (isosorbide) of the general formulae I b and I c

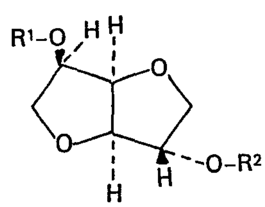

I b

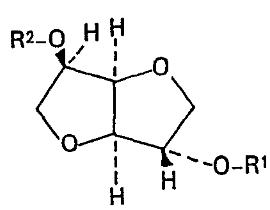

I c

in which $R^1$ and $R^2$ have the meaning as given in claim 1.

4. Acyl derivatives according to claim 1, characterized in that they are derivatives of 1,4:3,6-dianhydro-D-mannite (isomannide) of the general formula I d

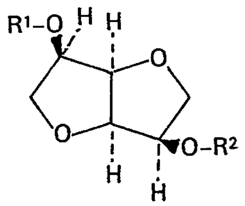

I d

in which $R^1$ and $R^2$ have the meaning as given in claim 1.

5. 5-[1,4-dihydro-2,6-dimethyl-3-methoxycarbonyl-4-(3-nitro-phenyl)-5-pyridylcarbonyl]-isosorbide

6. 5-[1,4-dihydro-2,6-dimethyl-3-methoxycarbonyl-4-(2-nitro-phenyl)-5-pyridylcarbonyl]-isosorbide

7. Process for the production of the acyl derivatives according to claim 1, characterized in that

A) an arylidene-β-ketocarboxylic acid ester of the general formula III

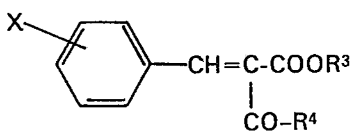

III

in which X, $R^3$ and $R^4$ have the meaning as given in claim 1, is reacted, in a generally known manner, with an enaminocarboxylic acid-(1,4:3,6-dianhydro-hexite)-ester of the general formula IV

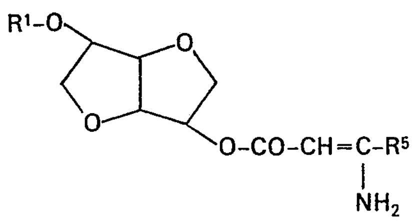

IV

in which $R^1$ and $R^5$ have the meaning as given in claim 1, or

B) an aldehyde of the general formula V

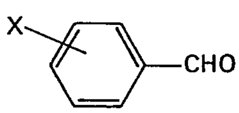

V

in which X has the meaning as given in claim 1, is reacted, in a generally known manner, with a β-ketocarboxylic acid ester of the general formula VI

$$R^4-CO-CH_2-COOR^3 \qquad VI$$

in which $R^3$ and $R^4$ have the meaning as given in claim 1 and with an enaminocarboxylic acid-(1,4:3,6-dianhydro-hexite)-ester of the general formula IV, in which $R^1$ and $R^5$ have the meaning as given in claim 1, or

C) an aldehyde of the general formula V, in which X has the meaning as given in claim 1, is reacted, in a generally known manner, with an enaminocarboxylic acid ester of the general formula VII

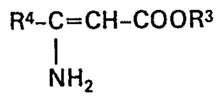

VII

in which $R^3$ and $R^4$ have the meaning as given in claim 1 and with a β-ketocarboxylic acid-(1,4:3,6-dianhydro-hexite)-ester of the general formula VIII

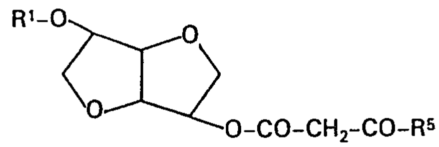

VIII

in which $R^1$ and $R^5$ have the meaning as given in claim 1, or

D) an arylidene-β-ketocarboxylic acid ester of the general formula III, in which X, $R^3$ and $R^4$ have the meaning as given in claim 1, is reacted, in a generally known manner, with a β-ketocarboxylic acid-(1,4:3,6-dianhydro-hexite)-ester of the general formula VIII, in which $R^1$ and $R^5$ have the meaning as given in claim 1, and with ammonia,

and, if desired, a thus obtained acyl derivative of the general formula I is converted to a salt in a generally known manner.

8. Process for the production of acyl derivatives according to claim 1, wherein in the general formula I $R^1$ means hydrogen, and $R^2$ has the meaning given in claim 1, characterized in that compounds of the general formula I, wherein $R^1$ means a lower acyl radical with 2 to 5 carbon atoms or a pyridylcarbonyl radical, and $R^2$ has the meaning as given in claim 1 is transesterified with a lower alcohol in a generally known manner or is subjected to a hydrolysis in the presence of acids or bases and, if desired, a thus obtained acyl derivative of the general formula I is converted to a salt in a generally known manner.

9. Process for the production of acyl derivatives according to claim 1, wherein in the general formula I $R^1$ means a lower acyl radical with 2 to 5 carbon atoms or a pyridylcarbonyl radical and $R^2$ has the meaning as given in claim 1, characterized in that compounds of the general formula I, wherein $R^1$ means hydrogen and $R^2$ has the meaning as given in claim 1 is acylated by means of a corresponding acid chloride or anhydride in a generally known manner and, if desired, a thus obtained acyl derivative of the general formula I is converted to a salt in a generally known manner.

10. Pharmaceutical preparations, characterized by a content of at least one compound as according to one of claims 1 to 6 as well as usual carriers and/or additives.

11. Pharmaceutical preparations for the treatment of circulatory diseases, characterized by a content of at least one compound as according to one of claims 1 to 6 as well as usual carriers and/or additives.

12. Acyl derivatives according to claim 3, characterized in that they have the formula Ib in claim 3, wherein $R^3$, $R^4$ and $R^5$ are each methyl.

**Revendications**

1. Dérivés acyliques de 1,4:3,6-dianhydrohexitols de formule générale I

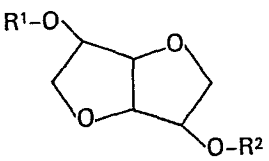

I

dans laquelle

$R^1$ représente l'hydrogène, un reste acyle inférieur ayant 2 à 5 atomes de carbone, un reste pyridylcarbonyle ou $NO_2$ et

$R^2$ représente un reste 1,4-dihydropyridyl-carbonyle de formule générale II

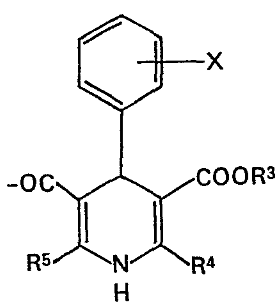

II

dans laquelle

X représente l'hydrogène ou 1, 2 ou 3 substituants identiques ou différents du groupe alkoxy en $C_1$ à $C_5$, alkyle en $C_1$ à $C_5$, cyano, di(alkyle en $C_1$ à $C_5$)amino, halogéno, nitro ou trifluorométhyle ou un groupe méthylène-dioxy,

$R^3$ est un reste hydrocarboné saturé ou non saturé à chaîne droite ou ramifié ayant 1 à 5 atomes de carbone, la chaîne pouvant être interrompue par un atome d'oxygène et/ou le reste hydrocarboné pouvant éventuellement être substitué par un groupe cyano,

$R^4$ et $R^5$ sont identiques ou différents et représentent chacun un groupe alkyle en $C_1$ à $C_5$, ainsi que les sels pharmaceutiquement acceptables de composés capables de former un sel.

2. Dérivés acyliques suivant la revendication 1, caractérisés en ce qu'ils sont des dérivés du 1,4:3,6-dianhydro-L-iditol (iso-idide) de formule générale Ia

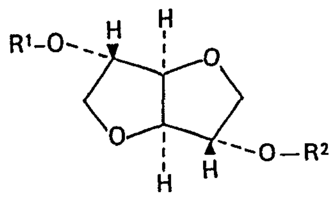

I a

dans laquelle $R^1$ et $R^2$ ont la définition indiquée dans la revendication 1.

3. Dérivés acyliques suivant la revendication 1, caractérisés en ce qu'ils sont des dérivés du 1,4:3,6-dianhydro-D-glucitol (isosorbide) de formules générales Ib et Ic

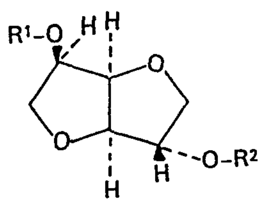

I b

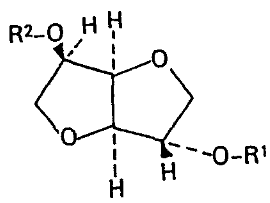

I c

dans lesquelles $R^1$ et $R^2$ ont la définition indiquée dans la revendication 1.

4. Dérivés acyliques suivant la revendication 1, caractérisés en ce qu'ils sont des dérivés du 1,4:3,6-dianhydro-D-mannitol (isomannide) de formule générale Id

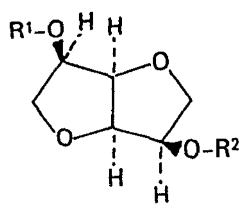

I d

dans laquelle $R^1$ et $R^2$ ont la définition indiquée dans la revendication 1.

5. Le 5-[1,4-dihydro-2,6-diméthyl-3-méthoxy-carbonyl-4-(3-nitrophényl)-5-pyridylcarbonyl]isosorbide.

6. Le 5-[1,4-dihydro-2,6-diméthyl-3-méthoxy-carbonyl-4-(2-nitrophényl)-5-pyridylcarbonyl]isosorbide.

7. Procédé de production des dérivés acyliques suivant la revendication 1, caractérisé en ce que

A) on fait réagir un ester d'acide arylidène-β-cétocarboxylique de formule générale III

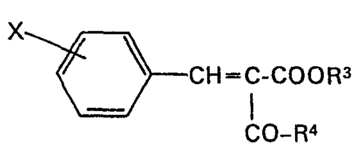

III

dans laquelle X, $R^3$ et $R^4$ ont la définition indiquée dans la revendication 1, d'une manière connue, avec un ester de 1,4:3,6-dianhydro-hexitol d'acide énamino-carboxylique de formule générale IV

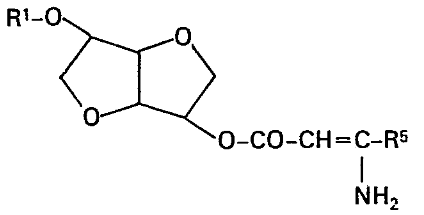

IV

dans laquelle $R^1$ à $R^5$ ont la définition indiquée dans la revendication 1, ou bien

B) on fait réagir un aldéhyde de formule générale V

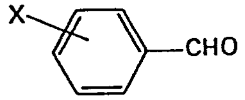

V

dans laquelle X a la définition indiquée dans la revendication 1, d'une manière connue avec un ester d'acide β-cétocarboxylique de formule générale VI

$R^4 - CO - CH_2 - COOR^3$ VI

dans laquelle $R^3$ et $R^4$ ont la définition indiquée dans la revendication 1 et avec un ester de 1,4:3,6-dianhydrohexitol d'acide énaminocarboxylique de formule générale IV dans laquelle $R^1$ et $R^5$ ont la définition indiquée dans la revendication 1, ou bien

C) on fait réagir un aldéhyde de formule générale V dans laquelle X a la définition indiquée dans la revendication 1, d'une manière connue avec un ester d'acide énaminocarboxylique de formule générale VII

$R^4-C=CH-COOR^3$ VII
|
$NH_2$

dans laquelle $R^3$ et $R^4$ ont la définition indiquée dans la revendication 1 et avec un ester de 1,4:3,6-dianhydrohexitol d'acide β-cétocarboxylique de formule générale VIII

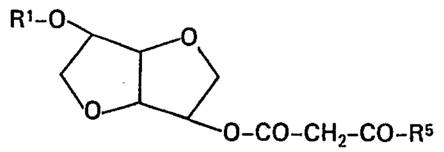

VIII

dans laquelle $R^1$ et $R^5$ ont la définition indiquée dans la revendication 1, ou bien

D) on fait réagir un ester d'acide arylidène-β-cétocarboxylique de formule générale III, dans laquelle X, $R^3$ et $R^4$ ont la définition indiquée dans la revendication 1, d'une manière connue avec un ester de 1,4:3,6-dianhydro-hexitol d'acide β-cétocarboxylique de formule générale VIII, dans laquelle $R^1$ et $R^5$ ont la définition indiquée dans la revendication 1 et avec l'ammoniac, et, le cas échéant, on transforme un dérivé acylique de formule générale I ainsi obtenu, d'une manière connue, en un sel.

8. Procédé de production de dérivés acyliques suivant la revendication 1, dans la formule générale desquels $R^1$ désigne l'hydrogène et $R^2$ a la définition indiquée dans la revendication 1, caractérisé en ce qu'on transestérifie d'une manière connue avec un alcool inférieur des composés de formule générale I dans laquelle $R^1$ est un reste acyle inférieur ayant 2 à 5 atomes de carbone ou un reste pyridylcarbonyle et $R^2$ a la définition indiquée dans la revendication 1, ou on les soumet à une hydrolyse en présence d'acides ou de bases, et, le cas échéant, on transforme en un sel d'une manière connue un dérivé acylique ainsi obtenu de formule générale I.

9. Procédé de production de dérivés acyliques suivant la revendication 1, dans la formule générale I desquels $R^1$ désigne un reste acyle inférieur ayant 2 à 5 atomes de carbone ou un reste pyridylcarbonyle et $R^2$ a la définition indiquée dans la revendication 1, caractérisé en ce qu'on acyle d'une manière connue au moyen d'un chlorure ou anhydride d'acide correspondant, des composés de formule générale I dans laquelle $R^1$ désigne l'hydrogène et $R^2$ a la définition indiquée dans la revendication 1 et, le cas échéant, on transforme en un sel d'une manière connue un dérivé acylique ainsi obtenu de formule générale I.

10. Préparations pharmaceutiques, caractérisées par une teneur en au moins un composé suivant l'une des revendication 1 à 6 ainsi qu'en excipients et/ou additifs classiques.

11. Préparations pharmaceutiques pour combattre des troubles circulatoires, caractérisées par une teneur en au moins un composé suivant l'une des revendications 1 à 6 ainsi qu'en excipients et/ou additifs classiques.

12. Dérivés acyliques suivant la revendication 3, caractérisés en ce qu'ils présentent la formule générale Ib suivant la revendication 3, dans laquelle $R^3$, $R^4$ et $R^5$ représentent chacun un groupe méthyle.